(19) Europäisches Patentamt / European Patent Office / Office européen des brevets

(11) **EP 3 618 840 B1**

(12) **EUROPEAN PATENT SPECIFICATION**

(45) Date of publication and mention
of the grant of the patent:
**27.12.2023   Bulletin 2023/52**

(21) Application number: **18723925.6**

(22) Date of filing: **03.05.2018**

(51) International Patent Classification (IPC):
*A61K 33/30* (2006.01)      *A61K 38/28* (2006.01)
*A61K 47/12* (2006.01)      *A61K 47/18* (2017.01)
*A61K 47/26* (2006.01)      *A61K 9/08* (2006.01)
*A61P 3/10* (2006.01)

(52) Cooperative Patent Classification (CPC):
(C-Sets available)
**A61K 33/30; A61K 9/0019; A61K 9/08;
A61K 38/28; A61K 47/12; A61K 47/183;
A61K 47/26; A61P 3/10**                    (Cont.)

(86) International application number:
**PCT/GB2018/051177**

(87) International publication number:
**WO 2018/203059 (08.11.2018 Gazette 2018/45)**

(54) **STABLE INSULIN FORMULATIONS**

STABILE INSULINFORMULIERUNGEN

FORMULATIONS D'INSULINE STABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **05.05.2017   GB 201707189**

(43) Date of publication of application:
**11.03.2020   Bulletin 2020/11**

(73) Proprietor: **Arecor Limited
Little Chesterford
Saffron Walden
CB10 1XL (GB)**

(72) Inventors:
 • **JEZEK, Jan
   Saffron Walden
   CB10 1XL (GB)**
 • **GERRING, David
   Saffron Walden
   CB10 1XL (GB)**
 • **HOWELL, Sarah
   Saffron Walden
   CB10 1XL (GB)**

 • **ZAKRZEWSKI, Leon
   Saffron Walden
   CB10 1XL (GB)**

(74) Representative: **Sagittarius IP
Marlow International
Parkway
Marlow, SL7 1YL (GB)**

(56) References cited:
**WO-A1-2012/006283      WO-A1-2015/120457
WO-A1-2017/191464      US-A1- 2009 137 455**

 • **RODERIKE POHL ET AL: "Ultra-Rapid
   Absorption of Recombinant Human Insulin
   Induced by Zinc Chelation and Surface Charge
   Masking Author Affiliations", JOURNAL OF
   DIABETES SCIENCE AND TECHNOLOGY, vol. 6,
   no. 4, 4 July 2012 (2012-07-04), pages 755-763,
   XP055206316,**

- **HADI MAHMOUDI MOGHADDAM ET AL: "Evaluation of Insulin Stability in the Presence of Nonionic Surface Active Agents (Polysorbate Groups) by Circular Dichroism and Fluorescence Spectroscopy", ASIAN JOURNAL OF BIOCHEMISTRY, vol. 10, no. 1, 1 January 2015 (2015-01-01), pages 17-30, XP55384029, ISSN: 1815-9923, DOI: 10.3923/ajb.2015.17.30**
- **LOUGHEED W D ET AL: "PHYSICAL STABILITY OF INSULIN FORMULATIONS", DIABETES, AMERICAN DIABETES ASSOCIATION, US, vol. 32, no. 5, 1 May 1983 (1983-05-01), pages 424-432, XP001096187, ISSN: 0012-1797, DOI: 10.2337/DIABETES.32.5.424**

Remarks:
  •The complete document including Reference Table(s) and the Sequence Listing(s) can be downloaded from the EPO website
  •The file contains technical information submitted after the application was filed and not included in this specification

(52) Cooperative Patent Classification (CPC): (Cont.)

C-Sets
**A61K 38/28, A61K 2300/00**

**Description**

FIELD OF THE INVENTION

This invention relates *inter alia* to rapid acting aqueous liquid formulations of insulin compounds as defined herein. Such formulations are suitable for the treatment of subjects suffering from diabetes mellitus, especially Type 1 diabetes mellitus.

BACKGROUND OF THE INVENTION

Diabetes mellitus ("diabetes") is a metabolic disorder associated with poor control of blood sugar levels leading to hypo or hyperglycemia. Untreated diabetes can lead to serious microvascular and macrovascular complications including coronary artery disease, peripheral artery disease, stroke, diabetic nephropathy, neuropathy and retinopathy. The two main types of diabetes are (i) Type 1 diabetes resulting from the pancreas not producing insulin for which the usual treatment is insulin replacement therapy and (ii) Type 2 diabetes where patients either produce insufficient insulin or have insulin resistance and for which treatments include insulin sensitising agents (such as metformin or pioglitazone), traditional insulin secretagogues (such as sulfonylureas), SGLT2 inhibitors (such as dapagliflozin, canagliflozin and empagliflozin) which reduce glucose absorption in the kidneys and so promote glucose excretion, GLP-1 agonists (such as exenatide and dulaglutide) which stimulate insulin release from pancreatic beta cells and DPPIV inhibitors (such as sitagliptin or vildagliptin) which inhibit breakdown of GLP-1 leading to increased insulin secretion. Patients with Type 2 diabetes may eventually require insulin replacement therapy.

For patients requiring insulin replacement therapy, a range of therapeutic options are possible. The use of recombinant human insulin has in recent times been overtaken by use of insulin analogues which have modified properties, for example, are longer acting or faster acting than normal insulin. Thus, a common regimen for a patient involves receiving a long acting basal insulin supplemented by a rapid acting insulin around mealtimes.

Insulin is a peptide hormone formed of two chains (A chain and B chain, respectively 21 and 30 amino acids in length) linked via disulfide bridges. Insulin normally exists at neutral pH in the form of a hexamer, each hexamer comprising three dimers bound together by zinc ions. Histidine residues on the insulin are known to be involved in the interaction with the zinc ions. Insulin is stored in the body in the hexameric form but the monomer form is the active form. Traditionally, therapeutic compositions of insulin have also been formulated in hexameric form in the presence of zinc ions. Typically, there are approximately three zinc cations per one insulin hexamer. It has been appreciated that the hexameric form is absorbed from the injection site considerably more slowly than the monomeric and dimeric form. Therefore, a faster onset of insulin action can be achieved if the hexameric form is destabilised allowing a more rapid dissociation of the zinc-bound hexamer into dimers and monomers in the subcutaneous space following injection. Three insulin analogues have been genetically engineered with this principle in mind. A first is insulin lispro (Humalog®) in which residues 28 and 29 of the B chain (Pro and Lys respectively) are reversed, a second is insulin aspart (NovoLog®) in which residue 28 of the B chain, normally Pro, is replaced by Asp and a third is insulin glulisine (Apidra®) in which residue 3 of the B chain, normally Asn, is replaced by Lys and residue 29 of the B chain, normally Lys, is replaced by Glu.

Whilst the existing rapid acting insulin analogues can achieve a more rapid onset of action, it has been appreciated that an even more rapid acting ("ultra rapid acting") insulins can be achieved by removing the zinc cations from insulin altogether. Unfortunately, the consequence of the hexamer dissociation is typically a considerable impairment in insulin stability both with respect to physical stability (e.g. stability to aggregation) and chemical stability (e.g. stability to deamidation). For example, monomeric insulin or insulin analogues having a rapid onset of action are known to aggregate and become physically unstable very rapidly because the formulation of insoluble aggregates proceeds via monomers of insulin. Various approaches to addressing this problem have been described in the art:

> US5,866,538 (Norup) describes insulin preparations of superior chemical stability comprising human insulin or an analogue or derivative thereof, glycerol and/or mannitol and 5 to 100mM of a halogenide (e.g. NaCl).
> US7,205,276 (Boderke) addresses the stability problems associated with preparing zinc free formulations of insulin and insulin derivatives and analogues and describes an aqueous liquid formulation comprising at least one insulin derivative, at least one surfactant, optionally at least one preservative and optionally at least one of an isotonicizing agent, a buffer and an excipient, wherein the formulation is stable and free from or contains less than 0.4% (e.g. less than 0.2%) by weight of zinc based on the insulin content of the formulation. The preferred surfactant appears to be polysorbate 20 (polyoxyethylene (20) sorbitan monolaurate).
> US2008/0194461 (Maggio) describes formulations of peptides and polypeptides including insulin which contain an alkylglycoside, which component is said to reduce aggregation and immunogenicity.
> WO2012/006283 (Pohl) describes formulations containing insulin together with a zinc chelator such as ethylenediaminetetraacetate (EDTA). Modulating the type and quantity of EDTA is said to change the insulin absorption profile.

Calcium EDTA is the preferred form of EDTA since it is said to be associated with reduced pain at the injection site and is less likely to remove calcium from the body. Preferred formulations also contain citrate which is said to further enhance absorption and to improve the chemical stability of the formulation.

US2010/0227795 (Steiner) describes a composition comprising insulin, a dissociating agent such as citric acid or sodium citrate, and a zinc chelator such as EDTA wherein the formulation has a physiological pH and is a clear aqueous solution. The formulations are said to have improved stability and rapid onset of action.

WO2015/120457 (Wilson) describes stabilized ultra-rapid acting insulin formulations comprising insulin in combination with a zinc chelator such as EDTA, a dissolution/stabilization agent such as citric acid, a magnesium salt, a zinc compound and optionally additional excipients.

Further approaches to accelerating the absorption and effect of insulin through the use of specific accelerating additives have been described:

WO91/09617 (Jorgensen) reports that nicotinamide or nicotinic acid or a salt thereof increases the speed of absorption of insulin from aqueous preparations administered parenterally.

WO2010/149772 (Olsen) describes a formulation comprising insulin, a nicotinic compound and arginine. The presence of arginine is said to improve the chemical stability of the formulation.

WO2015/171484 (Christe) describes rapid acting formulations of insulin wherein onset of action and/or absorption of insulin is faster due to the presence of treprostinil.

US2013/0231281 (Soula) describes an aqueous solution composition comprising insulin or an insulin analogue and at least one oligosaccharide whose average degree of polymerisation is between 3 and 13 and whose polydispersity index is above 1.0, said oligosaccharide having partially substituted carboxyl functional groups, the unsubstituted carboxyl functional groups being salifiable. Such a formulation is said to be rapid acting.

WO2017/191464 (Arecor Limited) describes formulations comprising insulin or an insulin analogue, ionic zinc, a chelating agent and polysorbate 80.

US2009/137455 (Steiner) describes a formulation containing a combination of a rapid acting insulin and a long acting insulin, wherein the pH of the rapid acting insulin is adjusted so that the long acting insulin remains soluble when they are mixed together.

Pohl et al. (Journal of Diabetes Science and Technology, vol. 6, no. 4, 2012, pages 755-763) describes *in vitro* experiments investigating a formulation of recombinant human insulin containing citrate and disodium ethylenediaminetetraacetic acid (EDTA).

Moghaddam et al. (Asian Journal of Biochemistry, vol. 10, no. 1, 2015, pages 17-30) describes a study investigating the possible interactions of insulin with four non-ionic polysorbate surfactants using circular dichroism and fluorescence spectroscopy.

Lougheed et al. (Diabetes, American Diabetes Association, US, vol. 32, no. 5, 1983, pages 424-432) describes a study investigating the effects of various compounds on the aggregation behaviour of crystalline zinc insulin (CZI) solutions.

It would be desirable if analogues or formulations of insulin were available which were ultra-rapid acting, thus more closely matching the activity of physiological insulin. There also remains a need in the art to provide further, and preferably improved, formulations of insulin and insulin analogues which are rapid acting and stable.

SUMMARY OF THE INVENTION

According to the invention there is provided an aqueous liquid pharmaceutical formulation comprising: (i) an insulin compound selected from insulin lispro, insulin aspart, insulin glulisine, and recombinant human insulin; (ii) ionic zinc; (iii) a zinc binding species at a concentration of 1 mM or more selected from species having a logK with respect to zinc ion binding in the range 4.5-10 at 25 °C, wherein said zinc binding species is citrate; (iv) a zinc binding species selected from species having a logK with respect to zinc ion binding of more than 12.3 at 25 °C at a concentration of between about 0.02 mM and about 0.2 mM; and (v) a non-ionic surfactant which is an alkyl glycoside selected from the group consisting of dodecyl maltoside, dodecyl glucoside, octyl glucoside, octyl maltoside, decyl glucoside, decyl maltoside, tridecyl glucoside, tridecyl maltoside, tetradecyl glucoside, tetradecyl maltoside, hexadecyl glucoside, hexadecyl maltoside, sucrose monooctanoate, sucrose mono decanoate, sucrose monododecanoate, sucrose monotridecanoate, sucrose monotetradecanoate and sucrose monohexadecanoate ("the formulation of the invention").

The formulations of the invention provide insulin in a form which is rapid or ultra rapid acting with good physical and chemical stability. As noted in the background discussion above, use of EDTA to chelate zinc ions in hexameric insulin does increase the rapidity of action but at the cost of greatly reduced stability. The present inventors have appreciated that a combination of a species which binds zinc less strongly, together with a small amount of a strong chelator such as EDTA and a non-ionic surfactant can achieve similar effects in terms of speed of action, but with much better stability.

Formulations of the invention may be used in treatment of subjects suffering from diabetes mellitus, particularly Type 1 diabetes mellitus especially for administration at meal times.

As can be seen from the accompanying examples, formulations of the invention are significantly more stable than corresponding formulations without non-ionic surfactant. The formulations are expected to be more rapidly acting than corresponding formulations which do not contain a zinc binding species. The inclusion of a small amount of a strong zinc binding species formulation in the presence of a non-ionic surfactant is believed to further increase the speed of action of insulin beyond that which is achieved by the weaker zinc binding species alone without compromising the stability of the formulation.

DESCRIPTION OF THE SEQUENCE LISTING

SEQ ID NO: 1: A chain of human insulin
SEQ ID NO: 2: B chain of human insulin
SEQ ID NO: 3: B chain of insulin lispro
SEQ ID NO: 4: B chain of insulin aspart
SEQ ID NO: 5: B chain of insulin glulisine

DETAILED DESCRIPTION OF THE INVENTION

As used herein, "insulin compound" refers to insulin and insulin analogues. The insulin compound in the appended claim set is selected from insulin lispro, insulin aspart, insulin glulisine, and recombinant human insulin.

As used herein, "insulin" refers to native human insulin having an A chain and a B chain as set out in SEQ ID NOs. 1 and 2 and containing and connected by disulfide bridges as in the native molecule (Cys A6-Cys A11, Cys B7 to Cys A7 and Cys-B19-Cys A20). Insulin is suitably recombinant insulin.

"Insulin analogue" refers to an analogue of insulin which is an insulin receptor agonist and has a modified amino acid sequence, such as containing 1 or 2 amino acid changes in the sequence of the A or B chain (especially the B chain). Desirably such amino acid modifications are intended to reduce affinity of the molecule for zinc and thus increase speed of action. Exemplary insulin analogues include faster acting analogues such as insulin lispro, insulin aspart and insulin glulisine. These forms of insulin have the human insulin A chain but variant B chains - see SEQ ID NOs. 3-5. Further faster acting analogues are described in EP0214826, EP0375437 and EP0678522. Thus, desirably an insulin analogue has a speed of action which is the same as or preferably greater than that of insulin. The speed of action of insulin or an insulin analogue may be determined in the Diabetic Pig Pharmacokinetic/Pharmacodynamic Model (see Examples, General Methods).

In one embodiment the insulin compound is recombinant human insulin. In another embodiment it is insulin lispro. In another embodiment it is insulin aspart. In another embodiment it is insulin glulisine.

The term "aqueous pharmaceutical formulation", as used herein, refers to a formulation suitable for therapeutic use in which the aqueous component is or comprises water, preferably distilled water, deionized water, water for injection, sterile water for injection or bacteriostatic water for injection. The aqueous pharmaceutical formulations of the invention are solution formulations in which all components are dissolved in water.

The concentration of insulin compound in the formulation will typically be in the range 10-1000 U/ml, such as 50-500 U/ml e.g. 50-200 U/ml. An exemplary formulation contains insulin compound at a concentration of 100 U/ml (around 3.6 mg/ml). Another range of interest is 500-1000 U/ml e.g. 800-1000 U/ml and another exemplary formulation contains insulin compound at a concentration of 1000 U/ml (around 36 mg/ml).

The formulations of the invention contain ionic zinc i.e. $Zn^{2+}$ ions. The source of the ionic zinc will typically be a water soluble zinc salt such as $ZnCl_2$, $ZnO$, $ZnSO_4$, $Zn(NO_3)_2$ or $Zn(acetate)_2$ and most suitably $ZnCl_2$ or $ZnO$.

The concentration of the ionic zinc in the formulation will typically be more than 0.05% e.g. more than 0.1% e.g. more than 0.2%, more than 0.3% or more than 0.4% by weight of zinc based on the weight of insulin compound in the formulation. Thus the concentration of the ionic zinc in the formulation may be more than 0.5% by weight of zinc based on the weight of insulin compound in the formulation, for example 0.5-1 %, e.g. 0.5-0.75%, e.g. 0.5-0.6% by weight of zinc based on the weight of insulin compound in the formulation. For the purpose of the calculation the weight of the counter ion to zinc is excluded.

In a formulation e.g. containing 100 U/ml of insulin compound the concentration of the ionic zinc will typically be more than 0.015 mM e.g. more than 0.03 mM e.g. more than 0.06 mM, more than 0.09 mM or more than 0.12 mM. Thus concentration of the ionic zinc in the formulation may be more than 0.15 mM, for example 0.15-0.60 mM, e.g.

0.20-0.45 mM, e.g. 0.25-0.35 mM.

In a formulation e.g. containing 1000 U/ml of insulin compound the concentration of the ionic zinc will typically be more than 0.15 mM e.g. more than 0.3 mM e.g. more than 0.6 mM, more than 0.9 mM or more than 1.2 mM. Thus concentration of the ionic zinc in the formulation may be more than 1.5 mM, for example 1.5-6.0 mM, e.g. 2.0-4.5 mM, e.g. 2.5-3.5 mM.

The formulations of the invention contain at least two different zinc binding species: a zinc binding species having a logK with respect to zinc ion binding in the range 4.5-10 at 25 °C, and a zinc binding species having a logK with respect to zinc ion binding of more than 12.3 at 25 °C. Metal binding stability constants listed in the National Institute of Standards and Technology reference database 46 (Critically Selected Stability Constants of Metal Complexes) can be used. The database typically lists logK constants determined at 25 °C. Therefore, the suitability zinc binding species for the present invention can be determined based on their logK metal binding stability constant with respect to zinc binding, as measured at 25 °C and as quoted by the database.

*Zinc binding species having a logK with respect to zinc ion binding in the range 4.5-10 at 25 °C*

The zinc binding species having a logK with respect to zinc ion binding in the range 4.5-10 at 25 °C is citrate (logK = 4.93) which can, for example, be employed as trisodium citrate.

The most suitable concentration of the zinc binding species will typically be in the range 1-100 mM.

For example, the concentration of the zinc binding species having a logK with respect to zinc binding in the range 4.5-10 at 25 °C in the formulation may typically be in the range 1-50 mM, more preferably 5-50 mM e.g. 10-50 mM e.g. 10-30 mM, more preferably around 20 mM (e.g. 22 mM), and especially for insulin compound 100 U/ml formulations. Suitably the concentration of the zinc binding species in the formulation is 10-50 mM e.g. 30-50 mM e.g. 40-50 mM, more preferably around 44 mM for insulin compound 1000 U/ml formulations.

In an embodiment, the concentration of the zinc binding species is 10 mM or more.

Anionic zinc binding species may be employed as the free acid or a salt form, such as a salt form with sodium or calcium ions, especially sodium ions.

Suitably the molar ratio of ionic zinc to zinc binding species having a logK with respect to zinc ion binding in the range 4.5-10 at 25 °C in the formulation is in the range 1:1 to 1:1000 e.g. 1:1 to 1:500 e.g. 1:3 to 1:500 e.g. 1:3 to 1:175.

For example, a suitable molar ratio of ionic zinc to zinc binding species is 1:10-1:500 e.g. 1:20-1:500 e.g. 1:20-1:100 or 1:40-1:250, e.g. 1:40-1:90 or 1:60-1:200, e.g. 1:60-1:80. The following ranges are particularly of interest: 1:10-1:500 e.g. 1:10-1:200 e.g. 1:10 to 1:100 e.g. 1:10-1:50, e.g. 1:10 to 1:30 (especially for insulin compound 1000 U/ml formulation) or 1:50-1:100, e.g. 1:60-1:80 (especially for insulin compound 100 U/ml formulation).

For example, a formulation containing 100 U/ml of insulin compound may contain around 0.3 mM of ionic zinc (i.e. around 19.7 $\mu$g/ml of ionic zinc, i.e. around 0.54% by weight of zinc based on the weight of insulin compound in the formulation) and around 15-30 mM e.g. 20-30 mM zinc binding species having a logK with respect to zinc ion binding in the range 4.5-10 at 25 °C.

For example, a formulation containing 1000 U/ml of insulin compound may contain around 3 mM of ionic zinc (i.e. around 197 $\mu$g/ml of ionic zinc, i.e. around 0.54% by weight of zinc based on the weight of insulin compound in the formulation) and around 30-60 mM e.g. 40-60 mM zinc binding species having a logK with respect to zinc ion binding in the range 4.5-10 at 25 °C.

Reference to citrate refers to the or an ionised form of the corresponding acid citric acid.

Citrate in acid form (citric acid) may be introduced into the aqueous formulations of the invention in the form of a salt of the acid, such as a sodium salt (sodium citrate). Alternatively, it can be introduced in the form of the acid with subsequent adjustment of pH to the required level.

*Zinc binding species having a logK with respect to zinc ion binding of more than 12.3 at 25 °C*

A preferred zinc binding species having a logK with respect to zinc ion binding of more than 12.3 at 25 °C is EDTA (logK = 14.5) which can, for example, be employed as disodium EDTA. A further example is EGTA (ethyleneglycoltetraacetate; logK = 12.6). Other possible zinc binding species may be selected from the following list:

Tetraethylenepentamine (logK = 15.1);
N-(2-hydroxyethyl)ethylenedinitrilotriacetate (HEDTA) (logK = 14.6);
1-Methyl-ethylenedinitrilotriacetate (PDTA) (logK = 17.5);
1-Ethyl-ethylenedinitrilotriacetate (logK = 18.3);
1-Propyl- thylenedinitrilotriacetate (logK = 18.2);
1-Carboxyethylene-ethylenedinitrilotriacetate (logK = 15.3);
Triethylenetetranitrilohexaacetate (TTHA) (logK = 17.9);

Tetraethylenepentanitriloheptaacetate (TPHA) (logK = 18.0); and
Tris(2-aminoethyl)amine (Tren) (logK = 14.5).

Typically said zinc binding species will have a logK with respect to zinc ion binding of 12.3-18 e.g. 12.3-16 at 25 °C.

The concentration of the zinc binding species having a logK with respect to zinc ion binding of more than 12.3 at 25 °C is between about 0.02 mM and about 0.2 mM.

For example, the concentration of the zinc binding species having a logK with respect to zinc ion binding of more than 12.3 at 25 °C in the formulation may typically be in the range about 0.02-0.15 mM e.g. 0.05-0.15 mM, more preferably about 0.1 mM, especially when the zinc binding species having a logK with respect to zinc ion binding of more than 12.3 at 25 °C is EDTA.

Anionic zinc binding species may be employed as the free acid or a salt form, such as a salt form with sodium or calcium ions, especially sodium ions.

A mixture of zinc binding species having a logK with respect to zinc ion binding of more than 12.3 at 25 °C may be employed, although a single zinc binding species is preferred.

Suitably the molar ratio of ionic zinc to zinc binding species having a logK with respect to zinc ion binding of more than 12.3 at 25 °C to ionic zinc in the formulation is in the range 1:1 to 1:100 e.g. 1:1 to 1:50 e.g. 1:2 to 1:25 or 1:4 to 1:20 e.g. 1:5 to 1:10, especially for EDTA as zinc binding species having a logK with respect to zinc ion binding of more than 12.3 at 25 °C.

For example, a formulation containing 100 U/ml of insulin compound may contain around 0.3 mM of ionic zinc (i.e. around 19.7 $\mu$g/ml of ionic zinc, i.e. around 0.54% by weight of zinc based on the weight of insulin compound in the formulation) and around 0.05-0.2 mM zinc binding species having a logK with respect to zinc ion binding of more than 12.3 at 25 °C (especially EDTA).

For example, a formulation containing 1000 U/ml of insulin compound may contain around 3 mM of ionic zinc (i.e. around 197 $\mu$g/ml of ionic zinc, i.e. around 0.54% by weight of zinc based on the weight of insulin compound in the formulation) and around 0.05-0.2 mM zinc binding species having a logK with respect to zinc ion binding of more than 12.3 at 25 °C (especially EDTA).

Suitably the molar ratio of citrate to zinc binding species having a logK with respect to zinc ion binding of more than 12.3 at 25 °C in the formulation is in the range 3:1 to 2000:1 e.g. 10:1 to 1500:1 e.g. 20:1 to 1000:1 or 50:1 to 1000:1 e.g. 100:1 to 500:1, especially for citrate as zinc binding species having a logK with respect to zinc ion binding in the range 4.5-10 at 25 °C and EDTA as zinc binding species having a logK with respect to zinc ion binding of more than 12.3 at 25 °C.

Without being limited by theory, the following is believed by the inventors to be relevant to the working of the invention: The logK is a measure of the strength of the coordinate bond between a ligand (i.e. zinc binding species in the context of the present invention) and a metal ion (i.e. ionic zinc in the context of the present invention). A ligand with higher logK will bind zinc more strongly than a ligand with lower logK and a skilled person will be able to calculate the equilibrium concentrations of ligand-metal complex(es), free ligand(s) and free metal(s) if logK constants and total concentrations of all ligands and metals are known. The hexameric structure of insulin can be dissociated in the presence of a species that has a sufficiently high logK with respect to zinc binding and is thus capable of removing zinc from the hexameric structure of insulin. Whilst a zinc binding species having a logK with respect to zinc binding of more than 12.3 will have the ability to dissociate the hexameric structure of insulin, a zinc binding species having a logK with respect to zinc binding in the range 4.5-10 will have a more limited (in some cases negligible) ability to dissociate the hexameric structure of insulin.

The formulations of the invention are preferably substantially free (e.g. less than 0.01 mM such as less than 0.005 mM and preferably free) of species having a logK with respect to zinc ion binding of 10-12.3 at 25 °C.

Zinc binding species which are acids (e.g. ethylenediaminetetraacetic acid) may be introduced into the aqueous solution in the form of a salt of the acid, such as a sodium salt (e.g. the disodium or tetrasodium salts of ethylenediaminetetraacetic acid). Alternatively, they can be introduced in the form of the acid with subsequent adjustment of pH to the required level.

The formulations of the invention contain a non-ionic surfactant which is an alkyl glycoside selected from dodecyl maltoside, dodecyl glucoside, octyl glucoside, octyl maltoside, decyl glucoside, decyl maltoside, tridecyl glucoside, tridecyl maltoside, tetradecyl glucoside, tetradecyl maltoside, hexadecyl glucoside, hexadecyl maltoside, sucrose monooctanoate, sucrose mono decanoate, sucrose monododecanoate, sucrose monotridecanoate, sucrose monotetradecanoate and sucrose monohexadecanoate, especially dodecyl maltoside.

The concentration of the non-ionic surfactant in the formulation will typically be in the range 1-1000 $\mu$g/ml, e.g. 5-500 $\mu$g/ml, e.g. 10-200 $\mu$g/ml, such as 10-100 $\mu$g/ml especially around 50 $\mu$g/ml.

Suitably the pH of the aqueous formulations of the invention is in the range 5.5-9.0 especially 6.5-8.0 e.g. 7.0-7.8. e.g. 7.0-7.5. In order to minimise injection pain the pH is preferably close to physiological pH (around pH 7.4). Another pH range of interest is 7.6-8.0 e.g. around 7.8.

Suitably, the formulation of the invention comprises a buffer in order to stabilise the pH of the formulation, which can also be selected to enhance protein stability. In one embodiment, a buffer is selected to have a pKa close to the pH of the formulation; for example histidine is suitably employed as a buffer when the pH of the formulation is in the range 5.0-7.0. Such a buffer may be employed in a concentration of 0.5-20 mM e.g. 2-5 mM. The citrate in the formulation as a zinc binding species may also have a buffering role. As another example, phosphate is suitably employed as a buffer when the pH of the formulation is in the range 6.1-8.1. Such a buffer may be employed in a concentration of 0.5-20 mM e.g. 2-5 mM. Alternatively, in another embodiment, the formulation of the invention is further stabilised as disclosed in WO2008/084237, which describes a formulation comprising a protein and one or more additives, characterised in that the system is substantially free of a conventional buffer, i.e. a compound with an ionisable group having a pKa within 1 unit of the pH of the formulation at the intended temperature range of storage of the formulation, such as 25 °C. In this embodiment, the pH of the formulation is set to a value at which the formulation has maximum measurable stability with respect to pH; the one or more additives (displaced buffers) are capable of exchanging protons with the insulin compound and have pKa values at least 1 unit more or less than the pH of the formulation at the intended temperature range of storage of the formulation. The additives may have ionisable groups having pKa between 1 to 5 pH units, preferably between 1 to 3 pH units, most preferably from 1.5 to 2.5 pH units, of the pH of the aqueous formulation at the intended temperature range of storage of the formulation (e.g. 25 °C). Such additives may typically be employed at a concentration of 0.5-10 mM e.g. 2-5 mM.

The aqueous formulations of the present invention cover a wide range of osmolarity, including hypotonic, isotonic and hypertonic formulations. Preferably, the formulations of the invention are substantially isotonic. Suitably the osmolarity of the formulation is selected to minimize pain according to the route of administration e.g. upon injection. Preferred formulations have an osmolarity in the range of about 200 to about 500 mOsm/L. Preferably, the osmolarity is in the range of about 250 to about 350 mOsm/L. More preferably, the osmolarity is about 300 mOsm/L.

Tonicity of the formulation may be adjusted with a tonicity modifying agent. Tonicity modifying agents may be charged or uncharged. Examples of charged tonicity modifying agents include salts such as a combination of sodium, potassium, magnesium or calcium ions, with chloride, sulfate, carbonate, sulfite, nitrate, lactate, succinate, acetate or maleate ions (especially sodium chloride or sodium sulphate, particularly sodium chloride). Amino acids such as arginine, glycine or histidine may also be used for this purpose. Charged tonicity modifying agent is preferably used at a concentration of 100 - 300 mM, e.g. around 150 mM. Examples of uncharged tonicity modifying agents include sugars, sugar alcohols and other polyols, such as trehalose, sucrose, mannitol, glycerol, 1,2-propanediol, raffinose, lactose, dextrose, sorbitol or lactitol (especially trehalose, mannitol, glycerol or 1,2-propanediol, particularly glycerol). Uncharged tonicity modifying agent is preferably used at a concentration of 200 - 500 mM, e.g. around 300 mM.

When the insulin compound is insulin lispro, the tonicity is suitably adjusted using an uncharged tonicity modifying agent, preferably at a concentration of 200 - 500 mM, e.g. around 300 mM. In this embodiment, the uncharged tonicity modifying agent is suitably selected from the group consisting of trehalose, mannitol, glycerol and 1,2-propanediol (most suitably glycerol). When the insulin compound is insulin aspart at a concentration of 500 U/ml or less (e.g. 100 U/ml), the tonicity is suitably adjusted using a charged tonicity modifying agent, especially sodium chloride, preferably at a concentration of 100 - 300 mM, e.g. around 150 mM. When the insulin compound is insulin aspart at a concentration of > 500 U/ml (e.g. 1000 U/ml), the tonicity is suitably adjusted using an uncharged tonicity modifying agent, preferably at a concentration of 200 - 500 mM, e.g. around 300 mM. In this embodiment, the uncharged tonicity modifying agent is suitably selected from the group consisting of trehalose, mannitol, glycerol and 1,2-propanediol (most suitably glycerol).

The ionic strength of a formulation may be calculated according to the formula:

$$I = 0.5 \times \sum_{X=1}^{n} c_x \, z_x^2$$

in which $c_x$ is molar concentration of ion x (mol L$^{-1}$), $z_x$ is the absolute value of the charge of ion x and the sum covers all ions (n) present in the formulation. The contribution of the insulin compound itself should be ignored for the purposes of the calculation. For zwitterions the absolute value of the charge is the total charge excluding polarity, e.g. for glycine the possible ions have absolute charge of 0, 1 or 2 and for aspartate the possible ions have absolute charge of 0, 1, 2 or 3.

In general, the ionic strength of the formulation is suitably in the range of around 5 mM up to around 500 mM.

When the insulin compound is insulin lispro, the ionic strength of the formulation is suitably kept to a minimum level since higher ionic strength formulations are less stable than lower ionic strength formulations. Suitably the ionic strength taking account of ions in the formulation except for the zinc binding species and the insulin compound is less than 40 mM, e.g. less than 20 mM, e.g. less than 10 mM such as 5-10 mM.

When the insulin compound is insulin aspart at a concentration of > 500 U/ml (e.g. 1000 U/ml), the ionic strength of the formulation is suitably kept to a minimum level since higher ionic strength formulations are less stable than lower ionic strength formulations. Suitably the ionic strength taking account of ions in the formulation except for the zinc binding species and the insulin compound is less than 40 mM, e.g. less than 20 mM, e.g. less than 10 mM.

When the insulin compound is insulin aspart at a concentration of 500 U/ml or less (e.g. 100 U/ml), the ionic strength of the formulation may be high. Suitably the ionic strength taking account of ions in the formulation except for the zinc binding species and the insulin compound is more than 50 mM, e.g. more than 100 mM, e.g. 50-500 mM or 100-500 mM or 100-300 mM such as around 150 mM.

The formulations of the invention can optionally include preservative, preferably phenol, m-cresol, chlorocresol, benzyl alcohol, propylparaben, methylparaben, benzalkonium chloride or benzethonium chloride.

The formulations of the invention may optionally comprise nicotinamide. The presence of nicotinamide may further increase the speed of onset of action of insulin formulated in formulations of the invention. Suitably, the concentration of nicotinamide is in the range 10-150 mM, preferably in the range 20-100 mM, such as around 80 mM.

The formulations of the invention may optionally comprise nicotinic acid or a salt thereof. The presence of nicotinic acid or a salt thereof may also further increase the speed of onset of action of insulin formulated in formulations of the invention. Suitably, the concentration of nicotinic acid or a salt thereof is in the range 10-150 mM, preferably in the range 20-100 mM, such as around 80 mM. Example salts include metal salts such as sodium, potassium and magnesium salts.

In an embodiment, one of nicotinamide and nicotinic acid (or as salt thereof) is included in the formulation but not both. In an embodiment, a mixture of nicotinamide and nicotinic acid (or as salt thereof) is included in the formulation.

Formulations of the invention may optionally include other beneficial components including stabilising agents. For example amino acids such as arginine or proline may be included which may have stabilising properties. Thus in one embodiment, the formulations of the invention comprise arginine.

Formulations of the invention may comprise a magnesium salt, such as magnesium chloride. Magnesium (as a salt) may typically be included at a concentration of 0.1 to 10 mM e.g. 1 to 5 mM such as around 4 mM. It has been reported that magnesium salts can reduce injection site irritation caused by EDTA (see WO2015/120457).

In an embodiment of the invention the formulations are free of acids selected from glutamic acid, ascorbic acid, succinic acid, aspartic acid, maleic acid, fumaric acid, adipic acid and acetic acid and are also free from the corresponding ionic forms of these acids.

In an embodiment of the invention the formulations are free of arginine.

In an embodiment of the invention the formulations are free of protamine and protamine salts.

In an embodiment of the invention the formulations are free of magnesium ions.

In an embodiment of the invention the formulations are free of calcium ions.

Suitably the formulations of the invention are sufficiently stable that the concentration of high molecular weight species remains low upon extended storage. The term "high molecular weight species" as used herein, refers to any irreversibly formed component of the protein content which has an apparent molecular weight at least about double the molecular weight of the parent insulin compound, as detected by a suitable analytical method, such as size-exclusion chromatography. That is, high molecular weight species are multimeric aggregates of the parent insulin compound. The multimeric aggregates may comprise the parent protein molecules with considerably altered conformation or they may be an assembly of the parent protein units in the native or near-native conformation. The determination of high molecular weight species can be done using methods known in the art, including size exclusion chromatography, electrophoresis, analytical ultracentrifugation, light scattering, dynamic light scattering, static light scattering and field flow fractionation.

Suitably the formulations of the invention are sufficiently stable that they remain substantially free of visible particles after storage at 30°C for at least one, two or three months. Visible particles are suitably detected using the 2.9.20. European Pharmacopoeia Monograph (Particulate Contamination: Visible Particles). For example, a formulation is substantially free of visible particles if it has a Visual score of 1 or 2, especially 1 according to the definition given in the Examples section.

Suitably the formulations of the invention are sufficiently stable that the concentration of related species remains low upon extended storage. The term "related species" as used herein, refers to any component of the protein content formed by a chemical modification of the parent insulin compound, particularly desamido or cyclic imide forms of insulin. Related species are suitably detected by RP-HPLC.

In a preferred embodiment, the formulation of the invention retains at least 95%, e.g. at least 96%, e.g. at least 97%, e.g. at least 98%, e.g. at least 99% parent insulin compound (by weight of total protein) after storage at 30°C for one, two or three months. The percentage of insulin compound (by weight of total protein) may be determined by size-exclusion chromatography or RP-HPLC.

In a preferred embodiment, the formulation of the invention comprises no more than 4% (by weight of total protein), preferably no more than 2% high molecular weight species after storage at 30°C for one, two or three months.

In a preferred embodiment, the formulation of the invention comprises no more than 4% (by weight of total protein), preferably no more than 2%, preferably no more than 1% A-21 desamido form of the insulin compound after storage at 30°C for one, two or three months.

In preferred embodiments, a formulation of the present invention should exhibit an increase in high molecular weight species during storage which is at least 10% lower, preferably at least 25% lower, more preferably at least 50% lower, than a formulation lacking the non-ionic surfactant but otherwise identical, following storage under the same conditions (e.g. 30°C) and length of time (e.g. one, two or three months).

In preferred embodiments, a formulation of the present invention should exhibit an increase in related species during storage which is at least 10% lower, preferably at least 25% lower, more preferably at least 50% lower, than a formulation lacking the non-ionic surfactant but otherwise identical, following storage under the same conditions (e.g. 30°C) and length of time (e.g. one, two or three months).

The speed of action of a formulation of the invention may be determined in the Diabetic Pig Pharmacokinetic/Pharmacodynamic Model (see Examples, General Methods). In preferred embodiments, a formulation of the present invention should exhibit a $T_{max}$ (i.e. time to peak insulin concentration) that is at least 10% shorter, preferably at least 20% shorter, more preferably at least 30% shorter than a formulation lacking the combination of zinc binding species but otherwise identical, using the model. In preferred embodiments, a formulation of the present invention should exhibit an area under the curve on the pharmacodynamics profile within the first 45 minutes after injection that is at least 10% greater, preferably at least 20% greater, more preferably at least 30% greater than a formulation lacking the combination of zinc binding species but otherwise identical, using the model.

According to further aspects of the invention, there is provided a formulation of the invention for use in the treatment of a subject suffering from diabetes mellitus.

A typical dose of the formulation of the invention is 2-30 U, e.g. 5-15 U. Administration should suitably occur in the window between 15 minutes before eating (i.e. before start of a meal) and 15 minutes after eating (i.e. after end of a meal).

A container e.g. made of plastics or glass may contain one dose or a plurality of doses of the formulation of the invention. The container can, for example, be a cartridge designed to be a replaceable item for use with an injection device.

The formulations of the invention may suitably be packaged for injection, especially sub-cutaneous or intramuscular injection. Sub-cutaneous injection is preferred. Injection may be by conventional syringe or more preferably via a pen device adapted for use by diabetic subjects. Exemplary pen devices include the Kwikpen® device and the Flexpen® device.

An injection device, particularly a device adapted for subcutaneous or intramuscular injection, for single or multiple use comprising a container containing one dose or a plurality of doses of the formulation of the invention together with an injection needle is also disclosed. In an embodiment the container is a replaceable cartridge which contains a plurality of doses. In an embodiment, the needle is replaceable e.g. after each occasion of use.

A medical device comprising a reservoir comprising plurality of doses of the formulation of the invention and a pump adapted for automatic or remote operation such that upon automatic or remote operation one or more doses of the formulation of the invention is administered to the body e.g. subcutaneously or intramuscularly is also disclosed. Such devices may be worn on the outside of the body or implanted in the body.

Formulations of the invention may be prepared by mixing the ingredients. For example, the insulin compound may be dissolved in an aqueous formulation comprising the other components. Alternatively, the insulin compound may be dissolved in a strong acid (typically HCl), after dissolution diluted with an aqueous formulation comprising the other components, and then pH adjusted to the desired pH with addition of alkali (e.g. NaOH). As a variation on this method, a step of neutralising the acid solution may be performed before the dilution step and it may then not be necessary to adjust the pH after the dilution step (or a small adjustment only may be necessary).

A dry solid pharmaceutical composition suitable for reconstitution with an aqueous medium which comprises (i) an insulin compound selected from insulin lispro, insulin aspart, insulin glulisine, and recombinant human insulin; (ii) ionic zinc e.g. at a concentration of 0.05% or more e.g. 0.5% or more by weight of zinc based on the weight of insulin compound in the formulation; (iii) citrate; (iv) a zinc binding species selected from species having a logK with respect to zinc ion binding of more than 12.3 at 25 °C at a concentration of between about 0.02 mM and about 0.2 mM; and (iv) a non-ionic surfactant which is an alkyl glycoside selected from the group consisting of dodecyl maltoside, dodecyl glucoside, octyl glucoside, octyl maltoside, decyl glucoside, decyl maltoside, tridecyl glucoside, tridecyl maltoside, tetradecyl glucoside, tetradecyl maltoside, hexadecyl glucoside, hexadecyl maltoside, sucrose monooctanoate, sucrose mono decanoate, sucrose monododecanoate, sucrose monotridecanoate, sucrose monotetradecanoate and sucrose monohexadecanoate, is disclosed.

Thus a formulation of the invention may be prepared by dissolving such a dry solid pharmaceutical composition in an aqueous medium e.g. water or saline. Such a dry solid pharmaceutical composition may be prepared by dehydrating (e.g. freeze drying) a formulation of the invention. Also provided is a container containing one dose or a plurality of doses of such a dry solid pharmaceutical composition.

Formulations of the invention are expected to have one or more of the following advantageous properties:

- rapid speed of action, typically faster than normal human insulin, upon administration to a subject;

- good physical stability upon storage, especially as measured by the amount of HMWS or visual detection of particles;
- good chemical stability upon storage, especially as measured by the amount of related products e.g. products of deamidation.

Abbreviations

| EDTA | ethylenediaminetetraacetate |
|------|------|
| EGTA | ethyleneglycoltetraacetate |
| HEDTA | N-(2-hydroxyethyl)ethylenedinitrilotriacetate |
| PDTA | 1-methyl-ethylenedinitrilotriacetate |
| TPHA | tetraethylenepentanitriloheptaacetate |
| Tren | tris(2-aminoethyl)amine |
| HPLC | high performance liquid chromatography |
| HMWS | high molecular weight species |
| RP | reverse phase |
| SEC | size-exclusion chromatography |

EXAMPLES

General Methods

(a) The Diabetic Pig Pharmacokinetic/Pharmacodynamic Model: Method for determining speed of action:

10 male diabetic Yucatan miniature pigs are used. Pigs are injected subcutaneously with a sample of the test formulation and blood is taken (1 or 2 ml) at the following time-points (min) with respect to the injection: -30 (or -15), 0, 5, 10, 15, 20, 30, 40, 50, 60, 75, 90, 105, 120, 150, 180, 210 and 240. For pharmacodynamics profile, serum is analysed for glucose (using a commercially available glucometer). For pharmacokinetic profile, insulin concentration is determined in the serum using an immunoassay.

(b) Visual assessment

Visible particles are suitably detected using the 2.9.20. European Pharmacopoeia Monograph (Particulate Contamination: Visible Particles). The apparatus required consists of a viewing station comprising:

- a matt black panel of appropriate size held in a vertical position
- a non-glare white panel of appropriate size held in a vertical position next to the black panel
- an adjustable lampholder fitted with a suitable, shaded, white-light source and with a suitable light diffuser (a viewing illuminator containing two 13 W fluorescent tubes, each 525 mm in length, is suitable). The intensity of illumination at the viewing point is maintained between 2000 lux and 3750 lux.

Any adherent labels are removed from the container and the outside washed and dried. The container is gently swirled or inverted, ensuring that air bubbles are not introduced, and observed for about 5 s in front of the white panel. The procedure is repeated in front of the black panel. The presence of any particles is recorded.

The visual scores are ranked as follows:

Visual score 1: >10 very small particles
Visual score 2: 10-20 very small particles
Visual score 3: 20-50 particles, including larger particles
Visual score 4: >50 particles, including larger particles

Whilst the particles in samples with visual scores 3 and 4 are clearly detectable on casual visual assessment under normal light, samples with visual score 1 and 2 generally appear as clear solutions on the same assessment. Samples with visual scores 1-2 are considered to be "Pass"; samples with visual score 3-4 are considered to be "Fail".

Example 1 - example formulations

The following example formulations may be prepared:

Example A:

| Insulin aspart | 100 U/ml |
| Sodium phosphate | 2 mM |
| phenol | 15.9 mM |
| m-cresol | 15.9 mM |
| Ionic zinc (as $ZnCl_2$) | 19.7 μg/ml (0.3 mM), equals 0.55% (w/w) based on the weight of insulin compound in the formulation |
| Citrate | 22 mM |
| NaCl | 150 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Example B:

| Insulin lispro | 100 U/ml |
| Sodium phosphate | 2 mM |
| phenol | 15.9 mM |
| m-cresol | 15.9 mM |
| Ionic zinc (as $ZnCl_2$) | 19.7 μg/ml (0.3 mM), equals 0.55% (w/w) based on the weight of insulin compound in the formulation |
| Citrate | 22 mM |
| Glycerol | 174 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Example C:

| Insulin aspart | 100 U/ml |
| Sodium phosphate | 2 mM |
| phenol | 15.9 mM |
| m-cresol | 15.9 mM |
| Ionic zinc (as $ZnCl_2$) | 19.7 μg/ml (0.3 mM), equals 0.55% (w/w) based on the weight of insulin compound in the formulation |
| Citrate | 22 mM |

(continued)

| NaCl | 150 mM |
|---|---|
| EDTA | 0.02 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Example D:

| Insulin lispro | 100 U/ml |
|---|---|
| Sodium phosphate | 2 mM |
| phenol | 15.9 mM |
| m-cresol | 15.9 mM |
| Ionic zinc (as $ZnCl_2$) | 19.7 $\mu$g/ml (0.3 mM), equals 0.55% (w/w) based on the weight of insulin compound in the formulation |
| Citrate | 22 mM |
| Glycerol | 174 mM |
| EDTA | 0.02 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Example E:

| Insulin aspart | 1000 U/ml |
|---|---|
| Sodium phosphate | 2 mM |
| phenol | 15.9 mM |
| m-cresol | 15.9 mM |
| Ionic zinc (as $ZnCl_2$) | 19.7 $\mu$g/ml (0.3 mM), equals 0.55% (w/w) based on the weight of insulin compound in the formulation |
| Citrate | 44 mM |
| Glycerol | 174 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Example F:

| Insulin lispro | 1000 U/ml |
|---|---|

(continued)

| Sodium phosphate | 2 mM |
|---|---|
| phenol | 15.9 mM |
| m-cresol | 15.9 mM |
| Ionic zinc (as $ZnCl_2$) | 19.7 $\mu$g/ml (0.3 mM), equals 0.55% (w/w) based on the weight of insulin compound in the formulation |
| Citrate | 44 mM |
| Glycerol | 174 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.4 | |

Example G:

| Insulin aspart | 100 U/ml |
|---|---|
| Sodium phosphate | 2 mM |
| phenol | 15.9 mM |
| m-cresol | 15.9 mM |
| Ionic zinc (as $ZnCl_2$) | 19.7 $\mu$g/ml (0.3 mM), equals 0.55% (w/w) based on the weight of insulin compound in the formulation |
| Citrate | 22 mM |
| NaCl | 150 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

Example H:

| Insulin lispro | 100 U/ml |
|---|---|
| Sodium phosphate | 2 mM |
| phenol | 15.9 mM |
| m-cresol | 15.9 mM |
| Ionic zinc (as $ZnCl_2$) | 19.7 $\mu$g/ml (0.3 mM), equals 0.55% (w/w) based on the weight of insulin compound in the formulation |
| Citrate | 22 mM |
| Glycerol | 174 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

Example I:

| | |
|---|---|
| Insulin aspart | 100 U/ml |
| Sodium phosphate | 2 mM |
| phenol | 15.9 mM |
| m-cresol | 15.9 mM |
| Ionic zinc (as $ZnCl_2$) | 19.7 μg/ml (0.3 mM), equals 0.55% (w/w) based on the weight of insulin compound in the formulation |
| Citrate | 22 mM |
| NaCl | 150 mM |
| EDTA | 0.02 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

Example J:

| | |
|---|---|
| Insulin lispro | 100 U/ml |
| Sodium phosphate | 2 mM |
| phenol | 15.9 mM |
| m-cresol | 15.9 mM |
| Ionic zinc (as $ZnCl_2$) | 19.7 μg/ml (0.3 mM), equals 0.55% (w/w) based on the weight of insulin compound in the formulation |
| Citrate | 22 mM |
| Glycerol | 174 mM |
| EDTA | 0.02 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

Example K:

| | |
|---|---|
| Insulin aspart | 1000 U/ml |
| Sodium phosphate | 2 mM |
| phenol | 15.9 mM |
| m-cresol | 15.9 mM |
| Ionic zinc (as $ZnCl_2$) | 19.7 μg/ml (0.3 mM), equals 0.55% (w/w) based on the weight of insulin compound in the formulation |
| Citrate | 44 mM |
| Glycerol | 174 mM |
| EDTA | 0.1 mM |
| Surfactant | dodecyl maltoside (0.05 mg/ml) |
| Water for injection | qs |

(continued)

| Residual NaCl | Acidification and subsequent neutralisation during preparation results in formation of 2-4 mM NaCl |
| pH adjusted to 7.8 | |

Example L:

| Insulin lispro | 1000 U/ml |
| Sodium phosphate | 2 mM |
| phenol | 15.9 mM |
| m-cresol | 15.9 mM |
| Ionic zinc (as $ZnCl_2$) | 19.7 $\mu$g/ml (0.3 mM), equals 0.55% (w/w) based on the weight of insulin compound in the formulation |
| Citrate | 44 mM |
| Glycerol | 174 mM |
| EDTA | 0.1 mM |
| Surfactant | dodecyl maltoside (0.05 mg/ml) |
| Water for injection | qs |
| Residual NaCl | Acidification and subsequent neutralisation during preparation results in formation of 2-4 mM NaCl |
| pH adjusted to 7.8 | |

*Method for preparation for the above formulations:*

Insulin powder is added to water and HCl is added until the powder is fully dissolved (pH has to be <3 in order to achieve full dissolution). $ZnCl_2$ is added to the required level. Once dissolved, pH is adjusted to approximately 7 and volume is adjusted with water so that the insulin concentration is $2\times$ the required concentration. The composition is then mixed 1:1 (v/v) with a mixture of additional excipients (all at $2\times$ the required concentration).

Example 2 - Stability of insulin aspart in the presence of low concentration of a strong chelating agent, with and without a surfactant

The effect of low concentration of EDTA on stability of insulin aspart was investigated both in the absence and in the presence of a surfactant. The effect was investigated in two different background solutions:

Background solution 1: sodium phosphate (13.2 mM), sodium citrate (9.3 mM), magnesium sulphate (4 mM), glycerol (173.7 mM), phenol (0.3 mM), m-cresol (29.1 mM), ionic zinc (19.7 $\mu$g/ml, as $ZnCl_2$), pH 7.4

Background solution 2: sodium phosphate (2 mM), sodium citrate (22 mM), sodium chloride (150 mM), phenol (15.9 mM), m-cresol (15.9 mM), ionic zinc (19.7 $\mu$g/ml, as $ZnCl_2$), pH 7.4

Composition of the background solution 1 is identical to that shown in WO2015/120457 application (formulation BIOD-288 in Table 8), except the concentration of EDTA.

The formulations tested are shown in Table 1.

Table 1: Additional components in formulations of insulin aspart tested.

| | Background solution | EDTA (mM) | Dodecyl-$\beta$-D-maltoside (mg/ml) |
| --- | --- | --- | --- |
| Formulation 1 | 1 | 0 | 0 |
| Formulation 2 | 1 | 0.02 | 0 |
| Formulation 3 | 1 | 0.05 | 0 |

(continued)

|  | Background solution | EDTA (mM) | Dodecyl-$\beta$-D-maltoside (mg/ml) |
|---|---|---|---|
| Formulation 4 | 1 | 0.1 | 0 |
| Formulation 5 | 1 | 0.2 | 0 |
| Formulation 6[1] | 1 | 0.33 | 0 |
| Formulation 7 | 2 | 0 | 0 |
| Formulation 8 | 2 | 0.02 | 0 |
| Formulation 9 | 2 | 0.05 | 0 |
| Formulation 10 | 2 | 0.1 | 0 |
| Formulation 11 | 2 | 0.2 | 0 |
| Formulation 12 | 2 | 0.33 | 0 |
| Formulation 13 | 2 | 0 | 0.05 |
| Formulation 14[2] | 2 | 0.02 | 0.05 |
| Formulation 15 | 2 | 0.05 | 0.05 |
| Formulation 16[3] | 2 | 0.1 | 0.05 |
| Formulation 17 | 2 | 0.2 | 0.05 |
| Formulation 18 | 2 | 0.33 | 0.05 |
| [1] corresponds to formulation BIOD-288 in Table 8 in WO2015/120457 [2] equivalent to formulation C in Example 1 [3] equivalent to formulation A in Example 1 | | | |

Stability of insulin aspart was tested by visual assessment. Results are shown in Table 2. Particle formation was observed in both background solution in the absence of EDTA and dodecyl-$\beta$-D-maltoside, reaching the "Fail" limit (Visual score 4) in 7 days. The presence of 0.02 mM EDTA resulted in no measurable difference. The presence of higher concentrations of EDTA (0.05 - 0.33 mM) resulted in acceleration of particle formation, the effect being proportional to EDTA concentration. The EDTA-containing formulations thus reached the "Fail" limit at earlier time-points. The presence of dodecyl-$\beta$-D-maltoside significantly delayed the particle formation. The formulations containing up to 0.2 mM EDTA in the presence of dodecyl-$\beta$-D-maltoside remained at the "Pass" level up to the 7 day time-point and only the formulation containing 0.33 mM EDTA reached the "Fail" limit.

Table 2: Visual scores of insulin aspart formulations following storage at 30 °C. Visual score 1: <10 very small particles; visual score 2: 10-20 very small particles; visual score 3: 20-50 particles, including larger particles; visual score 4: >50 particles, including larger particles.

|  | 0 weeks | 1 day | 4 days | 7 days | 14 days | 28 days |
|---|---|---|---|---|---|---|
| Formulation 1 | 1 | 1 | 1 | 3 | 4 | 4 |
| Formulation 2 | 1 | 1 | 1 | 3 | 4 | 4 |
| Formulation 3 | 1 | 1 | 3 | 3 | 4 | 4 |
| Formulation 4 | 1 | 1 | 3 | 3 | 4 | 4 |
| Formulation 5 | 1 | 1 | 4 | 4 | 4 | 4 |
| Formulation 6 | 1 | 1 | 4 | 4 | 4 | 4 |
| Formulation 7 | 1 | 1 | 1 | 3 | 3 | 4 |
| Formulation 8 | 1 | 1 | 1 | 3 | 4 | 4 |
| Formulation 9 | 1 | 1 | 3 | 3 | 4 | 4 |
| Formulation 10 | 1 | 2 | 3 | 4 | 4 | 4 |

(continued)

|  | 0 weeks | 1 day | 4 days | 7 days | 14 days | 28 days |
|---|---|---|---|---|---|---|
| Formulation 11 | 1 | 2 | 4 | 4 | 4 | 4 |
| Formulation 12 | 1 | 2 | 4 | 4 | 4 | 4 |
| Formulation 13 | 1 | 1 | 1 | 1 | 1 | 1 |
| Formulation 14 | 1 | 1 | 1 | 1 | 1 | 1 |
| Formulation 15 | 1 | 1 | 1 | 1 | 1 | 1 |
| Formulation 16 | 1 | 1 | 1 | 1 | 1 | 2 |
| Formulation 17 | 1 | 1 | 1 | 2 | 3 | 4 |
| Formulation 18 | 1 | 1 | 2 | 3 | 4 | 4 |

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The term "and/or" as used in a phrase such as "A and/or B" herein is intended to include both A and B; A or B; A (alone); and B (alone). Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

SEQUENCE LISTING

SEQ ID NO: 1: GIVEQCCTSICSLYQLENYCN
SEQ ID NO: 2: FVNQHLCGSHLVEALYLVCGERGFFYTPKT
SEQ ID NO: 3: FVNQHLCGSHLVEALYLVCGERGFFYTKPT
SEQ ID NO: 4: FVNQHLCGSHLVEALYLVCGERGFFYTDKT
SEQ ID NO: 5: FVKQHLCGSHLVEALYLVCGERGFFYTPET

**Claims**

1. An aqueous liquid pharmaceutical formulation comprising:

   (i) an insulin compound selected from insulin lispro, insulin aspart, insulin glulisine, and recombinant human insulin;
   (ii) ionic zinc;
   (iii) a zinc binding species at a concentration of 1 mM or more selected from species having a logK with respect to zinc ion binding in the range 4.5-10 at 25 °C, wherein said zinc binding species is citrate;
   (iv) a zinc binding species selected from species having a logK with respect to zinc ion binding of more than 12.3 at 25 °C at a concentration of between about 0.02 mM and about 0.2 mM; and
   (v) a non-ionic surfactant which is an alkyl glycoside selected from the group consisting of dodecyl maltoside, dodecyl glucoside, octyl glucoside, octyl maltoside, decyl glucoside, decyl maltoside, tridecyl glucoside, tridecyl maltoside, tetradecyl glucoside, tetradecyl maltoside, hexadecyl glucoside, hexadecyl maltoside, sucrose monooctanoate, sucrose mono decanoate, sucrose monododecanoate, sucrose monotridecanoate, sucrose monotetradecanoate and sucrose monohexadecanoate.

2. The formulation according to claim 1, wherein the insulin compound is present at a concentration of 10-1000 U/ml.

3. The formulation according to claim 1 or claim 2, wherein the ionic zinc is present at a concentration of more than 0.05% by weight of zinc based on the weight of insulin compound in the formulation;

preferably wherein the ionic zinc is present at a concentration of more than 0.5% by weight of zinc based on the weight of insulin compound in the formulation;

most preferably wherein the ionic zinc is present at a concentration of 0.5-1% by weight of zinc based on the weight of insulin compound in the formulation.

4. The formulation according to any one of claims 1 to 3, wherein the citrate is present at a concentration of 1-50 mM.

5. The formulation according to any one of claims 1 to 4, wherein the molar ratio of ionic zinc to citrate is 1:3 to 1:500.

6. The formulation according to any one of claims 1 to 5, wherein the alkyl glycoside is dodecyl maltoside.

7. The formulation according to any one of claims 1 to 6, wherein the non-ionic surfactant which is an alkyl glycoside is present at a concentration of 1-1000 $\mu$g/ml.

8. The formulation according to any one of claims 1 to 7, wherein the zinc binding species having a logK with respect to zinc ion binding of more than 12.3 at 25 °C is selected from ethylenediaminetetraacetate (EDTA), ethyleneglycoltetraacetate (EGTA), tetraethylenepentamine, N-(2-hydroxyethyl)ethylenedinitrilotriacetate (HEDTA), 1-methyl-ethylenedinitrilotriacetate (PDTA), 1-ethyl-ethylenedinitrilotriacetate, 1-propyl-thylenedinitrilotriacetate, 1-carboxyethylene-ethylenedinitrilotriacetate, triethylenetetranitrilohexaacetate, tetraethylenepentanitriloheptaacetate (TPHA) and tris(2-aminoethyl)amine (Tren).

9. The formulation according to claim 8, wherein the zinc binding species having a logK with respect to zinc ion binding of more than 12.3 at 25 °C is EDTA.

10. The formulation according to any one of claims 1 to 9, further comprising a tonicity modifying agent.

11. The formulation according to any of claims 1 to 10, further comprising a preservative; preferably wherein the preservative is selected from the group consisting of phenol, m-cresol, chlorocresol, benzyl alcohol, propylparaben, methylparaben, benzalkonium chloride and benzethonium chloride.

12. A formulation according to any one of claims 1 to 11, for use in the treatment of a subject suffering from diabetes mellitus.

13. An injection device for single or multiple use comprising a container containing one dose or a plurality of doses of the formulation according to any one of claims 1 to 12 together with an injection needle.

14. A medical device comprising a reservoir comprising plurality of doses of the formulation according to any one of claims 1 to 12 and a pump adapted for automatic or remote operation such that upon automatic or remote operation one or more doses of the formulation is administered to the body.

15. Use of a non-ionic surfactant which is an alkyl glycoside to improve the storage stability of an aqueous liquid pharmaceutical formulation comprising:

(i) an insulin compound selected from insulin lispro, insulin aspart, insulin glulisine, and recombinant human insulin;
(ii) ionic zinc;
(iii) a zinc binding species at a concentration of 1 mM or more selected from species having a logK with respect to zinc ion binding in the range 4.5-10 at 25 °C, wherein said zinc binding species is citrate; and
(iv) a zinc binding species selected from species having a logK with respect to zinc ion binding of more than 12.3 at 25 °C at a concentration of between about 0.02 mM and about 0.2 mM;

wherein the alkyl glycoside is selected from the group consisting of dodecyl maltoside, dodecyl glucoside, octyl glucoside, octyl maltoside, decyl glucoside, decyl maltoside, tridecyl glucoside, tridecyl maltoside, tetradecyl glucoside, tetradecyl maltoside, hexadecyl glucoside, hexadecyl maltoside, sucrose monooctanoate, sucrose mono decanoate, sucrose monododecanoate, sucrose monotridecanoate, sucrose monotetradecanoate and sucrose monohexadecanoate.

**Patentansprüche**

1. Wässrige, flüssige pharmazeutische Formulierung, umfassend:

   (i) eine Insulinverbindung ausgewählt aus Insulin Lispro, Insulin Aspart, Insulin Glulisin und rekombinantem Humaninsulin;
   (ii) ionisches Zink;
   (iii) eine Zink bindende Spezies in einer Konzentration von 1 mM oder mehr, ausgewählt aus Spezies mit einem logK in Bezug auf Zinkionenbindung im Bereich von 4,5-10 bei 25 °C, wobei die Zink bindende Spezies Citrat ist;
   (iv) eine Zink bindende Spezies, ausgewählt aus Spezies mit einem logK in Bezug auf Zinkionenbindung von mehr als 12,3 bei 25 °C in einer Konzentration zwischen etwa 0,02 mM und etwa 0,2 mM; und
   (v) ein nichtionisches Tensid, das ein Alkylglycosid ausgewählt aus der Gruppe bestehend aus Dodecylmaltosid, Dodecylglucosid, Octylglucosid, Octylmaltosid, Decylglucosid, Decylmaltosid, Tridecylglucosid, Tridecylmaltosid, Tetradecylglucosid, Tetradecylmaltosid, Hexadecylglucosid, Hexadecylmaltosid, Saccharosemonooctanoat, Saccharosemonodecanoat, Saccharosemonododecanoat, Saccharosemonotridecanoat, Saccharosemonotetradecanoat und Saccharosemonohexadecanoat ist.

2. Formulierung nach Anspruch 1, wobei die Insulinverbindung in einer Konzentration von 10-1.000 U/ml vorliegt.

3. Formulierung nach Anspruch 1 oder Anspruch 2, wobei das ionische Zink in einer Konzentration von mehr als 0,05 Gew.-% Zink, bezogen auf das Gewicht der Insulinverbindung in der Formulierung, vorliegt;

   wobei das ionische Zink vorzugsweise in einer Konzentration von mehr als 0,5 Gew.-% Zink, bezogen auf das Gewicht der Insulinverbindung in der Formulierung, vorliegt;
   wobei das ionische Zink am meisten bevorzugt in einer Konzentration von mehr als 0,5-1 Gew.-% Zink, bezogen auf das Gewicht der Insulinverbindung in der Formulierung, vorliegt.

4. Formulierung nach einem der Ansprüche 1 bis 3, wobei das Citrat in einer Konzentration von 1-50 mM vorliegt.

5. Formulierung nach einem der Ansprüche 1 bis 4, wobei das Molverhältnis von ionischem Zink zu Citrat 1:3 bis 1:500 beträgt.

6. Formulierung nach einem der Ansprüche 1 bis 5, wobei das Alkylglycosid Dodecylmaltosid ist.

7. Formulierung nach einem der Ansprüche 1 bis 6, wobei das nichtionische Tensid, das ein Alkylglycosid ist, in einer Konzentration von 1-1000 μg/ml vorliegt.

8. Formulierung nach einem der Ansprüche 1 bis 7, wobei die Zink bindende Spezies mit einem logK in Bezug auf Zinkionenbindung von mehr als 12,3 bei 25 °C aus Ethylendiamintetraacetat (EDTA), Ethylenglycoltetraacetat (EGTA), Tetraethylenpentamin, N-(2-Hydroxyethyl)ethylendiinitrilotriacetat (HEDTA), 1-Methylethylendinitrilotriacetat (PDTA), 1-Ethylethylendinitrilotriacetat, 1-Propylethylendinitrilotriacetat, 1-Carboxyethylenethylendinitrilotriacetat, Triethylentetranitrilohexaacetat, Tetraethylenpentanitriloheptaacetat (TPHA) und Tris(2-aminoethyl)amin (Tren) ausgewählt ist.

9. Formulierung nach Anspruch 8, wobei die Zink bindende Spezies mit einem logK in Bezug auf Zinkionenbindung von mehr als 12,3 bei 25 °C EDTA ist.

10. Formulierung nach einem der Ansprüche 1 bis 9, ferner umfassend ein Tonizitätsmodifizierungsmittel.

11. Formulierung nach einem der Ansprüche 1 bis 10, ferner umfassend ein Konservierungsmittel, wobei das Konservierungsmittel aus der Gruppe, bestehend aus Phenol, m-Cresol, Chlorcresol, Benzylalkohol, Propylparaben, Methylparaben, Benzalkoniumchlorid und Benzethoniumchlorid ausgewählt ist.

12. Formulierung nach einem der Ansprüche 1 bis 11 zur Verwendung bei der Behandlung eines Subjekts, das an Diabetes mellitus leidet.

13. Injektionsvorrichtung zur einmaligen oder mehrmaligen Verwendung, umfassend einen Behälter, der eine Dosis oder eine Vielzahl von Dosen der Formulierung nach einem der Ansprüche 1 bis 12 zusammen mit einer Injektionsnadel enthält.

14. Medizinische Vorrichtung, umfassend ein Reservoir, das eine Vielzahl von Dosen der Formulierung nach einem der Ansprüche 1 bis 12 umfasst, und eine Pumpe, die für automatischen oder ferngesteuerten Betrieb geeignet ist, sodass bei automatischem oder ferngesteuertem Betrieb eine oder mehrere Dosen der Formulierung an den Körper verabreicht werden.

15. Verwendung eines nichtionischen Tensids, das ein Alkylglycosid ist, zum Verbessern der Lagerstabilität einer wässrigen, flüssigen pharmazeutischen Formulierung, umfassend:

(i) eine Insulinverbindung ausgewählt aus Insulin Lispro, Insulin Aspart, Insulin Glulisin und rekombinantem Humaninsulin;
(ii) ionisches Zink;
(iii) eine Zink bindende Spezies in einer Konzentration von 1 mM oder mehr, ausgewählt aus Spezies mit einem logK in Bezug auf Zinkionenbindung im Bereich von 4,5-10 bei 25 °C, wobei die Zink bindende Spezies Citrat ist;
(iv) eine Zink bindende Spezies, ausgewählt aus Spezies mit einem logK in Bezug auf Zinkionenbindung von mehr als 12,3 bei 25 °C in einer Konzentration zwischen etwa 0,02 mM und etwa 0,2 mM;
(v) wobei das Alkylglycosid aus der Gruppe bestehend aus Dodecylmaltosid, Dodecylglucosid, Octylglucosid, Octylmaltosid, Decylglucosid, Decylmaltosid, Tridecylglucosid, Tridecylmaltosid, Tetradecylglucosid, Tetradecylmaltosid, Hexadecylglucosid, Hexadecylmaltosid, Saccharosemonooctanoat, Saccharosemonodecanoat, Saccharosemonododecanoat, Saccharosemonotridecanoat, Saccharosemonotetradecanoat und Saccharosemonohexadecanoat ausgewählt ist.

## Revendications

1. Formulation pharmaceutique liquide aqueuse comprenant :

(i) un composé insulinique choisi parmi l'insuline lispro, l'insuline asparte, l'insuline glulisine, et l'insuline humaine recombinante ;
(ii) le zinc ionique ;
(iii) une espèce de liaison au zinc à une concentration de 1 mM ou plus choisie parmi des espèces ayant un logK par rapport à la liaison à l'ion zinc dans la plage de 4,5 à 10 à 25 °C, dans laquelle ladite espèce de liaison au zinc est du citrate ;
(iv) une espèce de liaison au zinc choisie parmi des espèces ayant un logK par rapport à la liaison à l'ion zinc supérieur à 12,3 à 25 °C à une concentration comprise entre environ 0,02 mM et environ 0,2 mM ; et
(v) un tensioactif non ionique qui est un alkylglycoside choisi dans le groupe constitué du dodécyl maltoside, du dodécyl glucoside, de l'octyl glucoside, de l'octyl maltoside, du décyl glucoside, du décyl maltoside, du tridécyl glucoside, du tridécyl maltoside, du tétradécyl glucoside, du tétradécyl maltoside, de l'hexadécyl glucoside, de l'hexadécyl maltoside, du monooctanoate de saccharose, du monodécanoate de saccharose, du monododécanoate de saccharose, du monotridécanoate de saccharose, du monotétradécanoate de saccharose et du monohexadécanoate de saccharose.

2. Formulation selon la revendication 1, dans laquelle le composé insulinique est présent à une concentration de 10 à 1 000 U/ml.

3. Formulation selon la revendication 1 ou la revendication 2, dans laquelle le zinc ionique est présent à une concentration supérieure à 0,05 % en poids de zinc sur la base du poids de composé insulinique dans la formulation ;

de préférence dans laquelle le zinc ionique est présent à une concentration supérieure à 0,5 % en poids de zinc sur la base du poids de composé insulinique dans la formulation ;
de manière préférée entre toutes dans laquelle le zinc ionique est présent à une concentration de 0,5 à 1 % en poids de zinc sur la base du poids de composé insulinique dans la formulation.

4. Formulation selon l'une quelconque des revendications 1 à 3, dans laquelle le citrate est présent à une concentration de 1 à 50 mM.

5. Formulation selon l'une quelconque des revendications 1 à 4, dans laquelle le rapport molaire entre le zinc ionique et le citrate est de 1:3 à 1:500.

6. Formulation selon l'une quelconque des revendications 1 à 5, dans laquelle l'alkylglycoside est le dodécyl maltoside.

7. Formulation selon l'une quelconque des revendications 1 à 6, dans laquelle le tensioactif non ionique qui est un alkylglycoside est présent à une concentration de 1 à 1000 μg/ml.

8. Formulation selon l'une quelconque des revendications 1 à 7, dans laquelle l'espèce de liaison au zinc ayant un logK par rapport à la liaison aux ions zinc supérieur à 12,3 à 25 °C est choisie parmi l'éthylènediaminetétraacétate (EDTA), l'éthylèneglycoltétraacétate (EGTA), la tétraéthylènepentamine, le N-(2-hydroxyéthyl)éthylènedinitrilotriacétate (HEDTA), le 1-méthyl-éthylènedinitrilotriacétate (PDTA), le 1-éthyl-éthylènedinitrilotriacétate, le 1-propyl-thylènedinitrilotriacétate otriacétate, le 1-carboxyéthylène-éthylènedinitrilotriacétate, le triéthylènetétranitrilohexaacétate, le tétraéthylènepentanitriloheptaacétate (TPHA) et la tris(2-aminoéthyl)amine (Tren).

9. Formulation selon la revendication 8, dans laquelle l'espèce de liaison au zinc ayant un logK par rapport à la liaison à l'ion zinc supérieur à 12,3 à 25 °C est l'EDTA.

10. Formulation selon l'une quelconque des revendications 1 à 9, comprenant en outre un agent modificateur de tonicité.

11. Formulation selon l'une quelconque des revendications 1 à 10, comprenant en outre un conservateur ; de préférence dans laquelle le conservateur est choisi dans le groupe constitué du phénol, du m-crésol, du chlorocrésol, de l'alcool benzylique, du propylparabène, du méthylparabène, du chlorure de benzalkonium et du chlorure de benzéthonium.

12. Formulation selon l'une quelconque des revendications 1 à 11, destinée à être utilisée dans le traitement d'un sujet souffrant de diabète sucré.

13. Dispositif d'injection à usage unique ou multiple comprenant un récipient contenant une dose ou une pluralité de doses de la formulation selon l'une quelconque des revendications 1 à 12 avec une aiguille d'injection.

14. Dispositif médical comprenant un réservoir comprenant une pluralité de doses de la formulation selon l'une quelconque des revendications 1 à 12 et une pompe conçue pour un fonctionnement automatique ou à distance de sorte que, lors d'un fonctionnement automatique ou à distance, une ou plusieurs doses de la formulation sont administrées au corps.

15. Utilisation d'un tensioactif non ionique qui est un alkylglycoside pour améliorer la stabilité au stockage d'une formulation pharmaceutique liquide aqueuse comprenant :

(i) un composé insulinique choisi parmi l'insuline lispro, l'insuline asparte, l'insuline glulisine, et l'insuline humaine recombinante ;
(ii) le zinc ionique ;
(iii) une espèce de liaison au zinc à une concentration de 1 mM ou plus choisie parmi des espèces ayant un logK par rapport à la liaison à l'ion zinc dans la plage de 4,5 à 10 à 25 °C, dans laquelle ladite espèce de liaison au zinc est du citrate ; et
(iv) une espèce de liaison au zinc choisie parmi des espèces ayant un logK par rapport à la liaison à l'ion zinc supérieur à 12,3 à 25 °C à une concentration comprise entre environ 0,02 mM et environ 0,2 mM ;

dans lequel l'alkylglycoside est choisi dans le groupe constitué du dodécyl maltoside, du dodécyl glucoside, de l'octyl glucoside, de l'octyl maltoside, du décyl glucoside, du décyl maltoside, du tridécyl glucoside, du tridécyl maltoside, du tétradécyl glucoside, du tétradécyl maltoside, de l'hexadécyl glucoside, de l'hexadécyl maltoside, du monooctanoate de saccharose, du monodécanoate de saccharose, du monododécanoate de saccharose, du monotridécanoate de saccharose, du monotétradécanoate de saccharose et du monohexadécanoate de saccharose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5866538 A, Norup
- US 7205276 B, Boderke
- US 20080194461 A, Maggio
- WO 2012006283 A, Pohl
- US 20100227795 A, Steiner
- WO 2015120457 A, Wilson
- WO 9109617 A, Jorgensen
- WO 2010149772 A, Olsen

- WO 2015171484 A, Christe
- US 20130231281 A, Soula
- WO 2017191464 A
- US 2009137455 A, Steiner
- EP 0214826 A
- EP 0375437 A
- EP 0678522 A
- WO 2008084237 A

**Non-patent literature cited in the description**

- **POHL et al.** *Journal of Diabetes Science and Technology,* 2012, vol. 6 (4), 755-763
- **MOGHADDAM et al.** *Asian Journal of Biochemistry,* 2015, vol. 10 (1), 17-30

- **LOUGHEED et al.** Diabetes. American Diabetes Association, 1983, vol. 32, 424-432